Europäisches Patentamt

European Patent Office

Office européen des brevets

(11) Publication number: **0 089 647**
**B1**

(12) **EUROPEAN PATENT SPECIFICATION**

(45) Date of publication of patent specification: **16.06.87**

(21) Application number: **83102711.5**

(22) Date of filing: **18.03.83**

(51) Int. Cl.⁴: **A 61 B 5/00, A 61 M 25/00**

(54) **Hollow viscus tonometry.**

(30) Priority: **22.03.82 US 360718**

(43) Date of publication of application:
**28.09.83 Bulletin 83/39**

(45) Publication of the grant of the patent:
**16.06.87 Bulletin 87/25**

(84) Designated Contracting States:
**CH DE FR GB IT LI**

(56) References cited:
**AU-B-2 739 777**
**DE-A-2 345 270**
**US-A-2 470 665**
**US-A-3 227 154**
**US-A-3 548 805**
**US-A-3 572 315**
**US-A-3 893 448**
**US-A-3 951 136**
**US-A-3 952 730**
**US-A-3 958 562**
**US-A-4 168 703**

(73) Proprietor: **FIDDIAN-GREEN, Richard G.**
**127 Brigham Hill Road**
**North Grafton, Massachusetts 01536 (US)**

(72) Inventor: **Fiddian-Green, Richard G.**
**Box M017 B 3912 Clinical Faculty Office Building**
**Ann Arbor Michigan (US)**

(74) Representative: **Sternagel, Hans-Günther, Dr.**
**et al**
**Patentanwälte Dr. Michael Hann Dr. H.-G.**
**Sternagel Sander Aue 30**
**D-5060 Bergisch Gladbach 2 (DE)**

(56) References cited:
**US-A-4 221 567**
**US-A-4 244 377**
**US-A-4 274 417**
**US-A-4 304 239**

Courier Press, Leamington Spa, England.

## Description

This invention relates to a catheter suitable for the introduction into hollow organs for hollow viscus tonometry.

Stress ulceration and intestinal ischemia are two serious problems that plague physicians involved in the management of patients in intensive care units. Intestinal ischemia, in particular, has an insidious onset and may not be detected until days after the intestine has become gangrenous. A delay in the diagnosis of intestinal ischemia may have devastating consequences for a patient. The availability of means for early diagnosis and management of patients with these problems would have immediate applicability in all intensive care units, especially where the procedure can be conveniently conducted with reasonable safety and reliability.

It has been established that a fall in the intramucosal pH may precede the development of stress ulceration. It has been found in the laboratory, that a fall in intramucosal pH also occurs within minutes of inducing intestinal ischemia in dogs. The fall in pH in intestinal mucosa, and hence the likelihood of stress ulceration or ischemia, can be reliably calculated from a $pCO_2$ (partial pressure of $CO_2$) in luminal fluid and the bicarbonate concentration in arterial blood. The method of calculating the pH in intestinal mucosal tissue, pursuant to principles of the invention, has been validated by direct measurements under a variety of conditions simulating clinical problems. A correlation coefficient in the order of 0.92 to 0.95 has been obtained in each of sixteen dogs. It will be readily recognized that the validity of the procedure is inherently extensible to humans.

To measure the $pCO_2$ in the lumen of the gut, it is necessary to obtain a sample of fluid that has been in contact with the wall of the gut for a certain time period, usually at least half an hour. It is difficult to aspirate fluid from the lumen of the gut with any consistency, for any fluid instilled into the lumen passes into distal and proximal regions. It is much easier to obtain samples from the stomach, but samples obtained from the stomach frequency contain foreign material that can damage a gas analyzer.

US—A—4,221,567 discloses a catheter comprising a hollow tube which is suitable for the introduction into an organ of interest and a walled sampling chamber on the end portion of the tube. The interior of the sampling chamber being in communication with the interior of the catheter tube so that aspirating liquid in the sampling chamber can be withdrawn from the chamber. The wall material of the sampling chamber is freely permeable to gas and poorly permeable to liquid.

It is the object of the invention to provide a new and unique catheter via which the desired sample or samples can be obtained without the complications of prior techniques. The object is solved by a catheter suitable for the introduction into hollow internal organs having a hollow catheter tube (12) being surrounded at its closed end by a sampling tubular element (18a) of a material which is freely permeable to gaseous fluid but poorly permeable to liquid fluid and which is in communication with the interior of the catheter tube (12), characterized in that the axial end segments of the tubular element (18a) are secured to the outer wall of the tube (12) thereby forming a sampling chamber (18) adjacent to the tube end (14), the wall material of the sampling chamber (18) has a certain elasticity so as to allow the enclosure to assume a slightly ballooned or ovoidal shape when filled by an aspirating liquid. The wall of the sampling chamber comprises a material which is substantially impermeable to liquid yet is highly permeable to gas. One suitable material is polydimethylsiloxane elastomer.

In use, the catheter is introduced into a patient to place the sampling chamber at a desired site within the organ of interest. An aspirating liquid fills the interior of the sampling chamber. The sampling chamber is left in place at the desired sampling site long enough to allow the gases present to diffuse through the wall of the sampling chamber into the aspirating liquid. The time should be long enough for the gases to equilibrate. The liquid impermeable nature of the sampling chamber wall material prevents both the aspirating liquid from leaking out of the chamber and also the intrusion of any liquids into the aspirating liquid. After the appropriate amount of placement time has elapsed, the aspirating liquid is aspirated along with the gases which have diffused into it. The sample thus obtained is analyzed for gas content, in particular for $pCO_2$. In this way, the $pCO_2$ within the lumen of the gut can be reliably measured with the fluid being free from lumenal debris.

In carrying out the diagnostic method the $pCO_2$ measurement is utilized in conjunction with a measurement of the bicarbonate concentration in an arterial blood sample of the patient for determining the pH of the tract wall.

Depending upon the particular condition of a given patient, the catheter may be left in place and samples may be taken at periodic intervals so that pH values may be periodically calculated. The procedure has a high reliability in accurately diagnosing intestinal ischemia in its incipient stages and such detection can be useful in treating the patient so that the potentially devastating consequences resulting from less timely detection may often be avoided.

The invention has applicability to many hollow internal organs although in the techniques described in detail herein, the catheter is particularly used for diagnosis within the gastrointestinal tract system. Depending upon the particular site or sites of interest within a patient, different types of catheters embodying principles of the invention may be appropriately used. One embodiment involves a catheter as described above. In that embodiment, the catheter has a single sampling chamber and a single walled tube. Another embodiment contemplates the structure of multiple individual single sampling chamber catheters of varying lengths bundled together to form a multiple sampling site catheter. Still another embodiment involves a catheter tube comprising a sump-type nasogastric tube. Yet another embodiment comprises a pliable catheter with a mercury bag at its end which may be used for certain procedures.

In use of an embodiment that employs multiple sampling chambers, the pH in intestinal mucosal tissue at one site may be calculated and compared with the calculated pH values at other sites. This analysis can be a useful diagnostic aid to the attending physician. In the case of an abdominal aortic resection, a multiple sampling chamber type catheter may be placed intralumenally in series in the colon at the time of the resection, and it may be used to aid in the early detection of colonic ischemia that occurs insidiously in approximately five percent of the patients subjected to this major operation. A multiple sampling chamber embodiment may also be introduced into the small intestine to monitor the pH and hence perfusion of the gut in patients with low flow states. In critically ill patients who require a nasogastric tube, a single sampling chamber embodiment may be incorporated into a conventional nasogastric tube and placed in the patient's stomach.

It is further contemplated that the invention may be practiced in connection with diagnosis of the billary tract, urinary tract and pancreas for monitoring pH and hence perfusion of the associated organs.

The foregoing features and benefits of the invention in its several aspects, along with additional features and benefits, will be seen in the ensuing description and claims which should be considered in conjunction with the accompanying drawings. The drawings disclose presently preferred embodients of catheters which embody principles of the invention and are used in the diagnostic aspects.

Brief Description of the Drawings:
FIG. 1 is a diagrammatic view of a catheter embodying principles of the invention.
FIG. 2 is another embodiment of catheter embodying principles of the invention.
FIG. 3 is yet another embodiment of catheter embodying principles of the invention.
FIG. 4 is still another embodiment of catheter embodying principles of the invention.

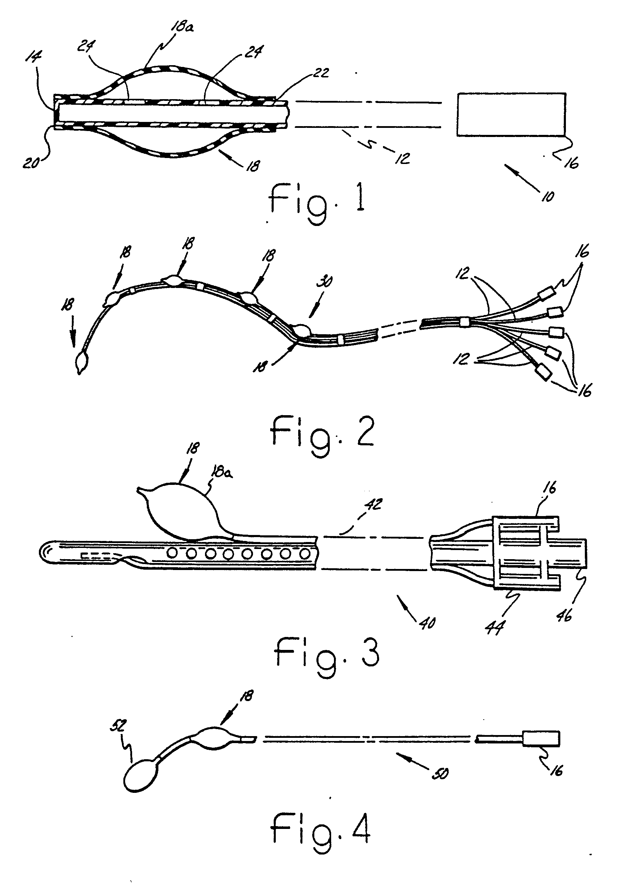

FIG. 1 illustrates a first embodiment of catheter 10. The catheter comprises a length of suitable tubing 12, one end 14 of which is closed, and the opposite end of which contains a connector such as a Luer-lock 16 or equivalent. A sampling chamber 18 is provided on the tube adjacent the closed end 14.

The illustrated embodiment utilizes a tubular element 18a forming the sampling chamber wall. The preferred form of tubular element is polydimethylsiloxane elastomer. The tubular element has an internal diameter which allows it to be fitted over the tubing 12. The axial end segments of the tubular element 18a are secured to the outer wall of tube 12 at the locations indicated by the reference numerals 20 and 22. The attachment may be made in any suitable fashion with adhesive being a suitable attachment medium. Thus, the ends of the tubular element 18a are sealed in a closed relationship to the outer wall of the tube 12 thereby forming the sampling chamber 18 adjacent tube end 14. The wall material of the sampling chamber has a certain elasticity so as to allow the enclosure to assume a slightly ballooned or ovoidal shape when filled by aspirating liquid, as will be explained hereinafter.

Before the tubular element 18a is inserted over tube 12, suitable apertures 24 (shown on an enlarged scale in the drawing) are provided in the wall of tube 12 so that after assembly of the tubular element 18a and the tube 12, the apertures 24 provide communication between the interior of tube 12 and the interior of the sampling chamber 18.

The material of the tubular element 18a possesses a characteristic whereby it is poorly permeable to liquid fluid while it is freely permeable to gaseous fluid. This property is important in practice of the invention. The material is also substantially impervious to the contents of the intestinal tract.

In one form of use, the catheter is introduced into a patient by being fed into the colon from the anus and positioned intraoperatively. A suitable aspirating fluid, such as a saline solution, is introduced via the Luer-lock 16, tube 12, and apertures 24 to fill the interior of the sampling chamber. The fluid passes through the apertures 24 filling the interior of the sampling chamber such that the sampling chamber assumes a balloon-like state.

In a form of use the catheter is placed such that the sampling chamber is at a desired sampling site in the internal organ of interest. It is left at this site for a sufficient amount of time to allow gases, carbon dioxide being the particular gas of interest, to diffuse across the wall of the chamber into the aspirating liquid. Desirably, the length of time should be sufficient to allow the gases to equilibrate. For example, one half hour may be suitable in certain applications.

The aspirating liquid containing the carbon dioxide gas is then withdrawn via the Luer end lock 16. The aspirated sample thus obtained is subjected to analysis by a conventional gas analyzer to measure the $pCO_2$ content of the lumenal fluid. A measurement of the bicarbonate concentration in the arterial blood of the patient is also obtained. These two measurements are then used to calculate the pH of the tract. Measurements may be taken at periodic intervals in the same manner and in this way a record of pH values can be established.

The recognition of the principle that the partial pressure of gas in the lumen of the gastrointestinal tract is the same or very close to that in the wall of the gastrointestinal tract under a steady state condition and hence, can be used as a measure of the partial pressure of gas, especially $CO_2$, in the wall of that part of the gastrointestinal tract. The pH in the wall of the gastrointestinal tract can be calculated from this value if the bicarbonate concentration in arterial blood is also known. With the catheter of the invention, the partial pressure of gas within the gastrointestinal tract can be readily measured because it allows a clear fluid sample, free of objectionable particulates and the like, to be obtained.

As explained earlier, a drop in the intramucosal pH has been found to accompany development of intestinal ischemia, and therefore the pH monitoring can be used to monitor for the incipiency of this potentially devastating condition. The earlier warning obtained with the invention offers the possibility of earlier treatment to counteract the condition.

FIG. 2 illustrates a further embodiment of the catheter 30. This embodiment is also useful in the colon. The catheter 30 comprises multiple sampling chambers 18 at spaced locations along the length of the catheter. In this regard, the catheter 30 is constructed as a bundle of individual catheters, such as the catheter 10 of FIG. 1, the individual catheters having various lengths. The illustrated example has five sampling chambers. This allows measurements to be taken at five different sites within the organ of interest and is useful for monitoring pH values not only in time at a particular sampling site but also in respect to concurrent pH measurements at different sites.

FIG. 3 illustrates a further embodiment of catheter 40 which comprises a tube 18a forming the wall of the sampling chamber; however, the tube 42 comprises a conventional double lumen nasogastric sump tube with a third lumen for the sampling chamber 18. The air and aspiration ports 44, 46 are of the nasogastric tube and the Luer-end lock 16 is for the third lumen which leads to the sampling chamber 18. The catheter 40 is intended for use in the stomach. In this regard, the catheter may be inserted into a patient in the same manner as a nasogastric tube, and the aspirating fluid for obtaining the $CO_2$ measurement is introduced and aspirated via Luer-lock 16 in the same manner as that for the previously described catheters.

FIG. 4 illustrates a still further embodiment of catheter 50 which is the same as the embodiment 10 of FIG. 1 except that the end 14 includes a sealed mercury bag 52. This catheter is intended for use in the small intestine, and it should be very soft and pliable with the mercury bag allowing peristalsis to position the tube in the small intestine. It should be long enough to reach the terminal ileum, and the same length as a colonscope would be more than adequate.

If desired, any of the embodiments of single catheter may be bundled together as in the manner of FIG. 2 so as to provide multiple sampling sites in any catheter construction.

Desirably, the volume of the sampling chamber should be relatively small in order to facilitate rapid equilibration of gas yet it must be large enough so that a suitable sample of about one milliliter for use in the gas analyzer can be withdrawn via the element 16. For example, around two milliliters is a suitable volume. The tubes such as the tube 12 should be of small caliber to insure as small a dead space as possible within the patient when in use. Tube 12 should also have as small a fluid volume (say about two-tenths milliliter) so that a minimum of aspirating liquid need be withdrawn at element 16 in advance of the sample from the chamber 18. The tube wall 12 should also be impermeable to gas. The Luer-end locks are conventional for connection to a syringe when aspirating fluid is to be introduced or withdrawn. The catheters may also contain rapid opaque markers for use in verifying position of the sampling chambers in the gut.

Where the catheter is to be left in the lumen of the gut for an extended period of time, for example, several days, it should be soft enough to be allowed to remain in this position without damage to the wall of the gut. To facilitate insertion, for example, into the colon, the catheter should be firm enough to allow for proper feeding. In this regard, it may be appropriate to use a wire stent during insertion to facilitate positioning of the catheter with the wire stent being removed after proper positioning has been obtained.

While the preferred embodiment has been disclosed in connection with monitoring of the gastrointestinal tract, it will be appreciated that its principles are applicable to other hollow internal organs to monitor pH and hence perfusion of those organs.

**Claims**

1. A catheter suitable for the introduction into hollow internal organs having a hollow catheter tube (12) being surrounded at its closed end by a sampling tubular element (18a) of a material which is freely permeable to gaseous fluid but poorly permeable to liquid fluid and which is in communication with the interior of the catheter tube (12), characterized in that the axial end segments of the tubular element (18a) are secured to the outer wall of the tube (12) thereby forming a sampling chamber (18) adjacent to the tube end (14), the wall material of the sampling chamber (18) has a certain elasticity so as to allow the enclosure to assume a slightly ballooned or ovoidal shape when filled by an aspirating liquid.

2. The catheter of Claim 1 characterized in that the catheter (30) comprises in addition to said catheter tube (12) and said sampling chamber (18) one or more additional catheter tubes (12) each of which has associated therewith a corresponding sampling chamber (18), all the tubes (12) comprising the catheter being of varying length, so that the respective sampling chambers (18) are disposed at different locations along the length of the catheter (30), the walls of all the sampling chambers (18) being of the same material.

3. The catheter of Claims 1 or 2, characterized in that the catheter tube (42) comprises a double lumen nasogastric sump tube with a third lumen for the sampling chamber (18) at the end of the third lumen.

4. The catheter of Claims 1 or 2, characterized in that the end (14) of the catheter tube (12) includes a sealed mercury bag (52).

5. The catheter of Claims 1 to 4, characterized in that the material of the sampling chamber (18) is polydimethylsiloxane elastomer.

**Patentansprüche**

1. Zur Einführung in Hohlräume innerer Organe geeigneter Katheter mit einem hohlen Katheterrohr (12), das an seinem geschlossenen Ende von einem rohrförmigen Probennahmeteil (18a) aus einem Material, das für Gase frei durchlässig, jedoch für Flüssigkeiten schwer durchlässig ist, umgeben ist, und das in Verbindung mit dem Innenraum des Katheterrohres (12) steht, dadurch gekennzeichnet, daß axiale Endsegmente des rohrförmigen Teils (18a) mit der Außenwand des Rohres (12) verbunden sind, so daß in Nachbarschaft zum Rohrende (14) eine Probennahmekammer (18) ausgebildet ist, daß das Wandmaterial der Probennahmekammer (18) eine gewisse Elastizität aufweist, so daß ihr Inhalt bei Füllung mit eingesaugter Flüssigkeit eine leicht ballonförmige oder eiförmige Form annimmt.

2. Katheter nach Anspruch 1, dadurch gekennzeichnet, daß der Katheter (30) zusätzlich zum Katheterrohr (12) und der Probennahmekammer (18) ein oder mehrere weitere Katheterrohre (12) aufweist, die jeweils mit einer korrespodierenden Probennahmekammer (18) verbunden sind, wobei alle Rohre (12) des Katheters unterschiedliche Länge aufweisen, so daß die betreffenden Probennahmekammern (18) an unterschiedlichen Stellen längs des Katheters (30) angeordnet sind und die Wände aller Probennahmekammern (18) aus dem gleichen Material sind.

3. Katheter nach Ansprüchen 1 oder 2, dadurch gekennzeichnet, daß das Katheterohr (42) ein doppellumiges nasogastrales Sammelrohr mit einem dritten Lumen für eine Probennahmekammer (18) am Ende des dritten Lumens aufweist.

4. Katheter nach Ansprüchen 1 oder 2, dadurch gekennzeichnet, daß das Ende (14) des Katheterrohrs (12) einen verschlossenen, Quecksilber enthaltenden Beutel (52) aufweist.

5. Katheter nach Ansprüchen 1 bis 4, dadurch gekennzeichnet, daß das Material der Probennahmekammer (18) Polydimethylsiloxanelastomer ist.

**Revendications**

1. Cathéter convenant pour l'introduction dans des organes internes creux, comprenant un tube creux de cathéter (12) étant entouré à son extrêmité fermée par un élément tubulaire de prélèvement d'échantillons (18a) en une matière, qui est aisément perméable aux fluides gazeux, mais perméable de manière médiocre aux fluides liquides, et qui est en communication avec l'intérieur du tube de cathéter (12), caractérisé en ce que les segments extrêmes de l'élément tubulaire (18a) dans le sens axial sont fixés sur la paroi extérieure du tube (12), formant ainsi une chambre de prélèvement d'échantillons adjacente à l'extrêmité (14) du tube, la matière de la paroi de cette chambre de prélèvement (18) présente une certaine élasticité de manière à permettre à l'enveloppe d'adopter une forme légèrement gonflée en ballon ou ovoïdale, lorsqu'elle est remplie par un liquide d'aspiration.

2. Cathéter de la revendication 1, caractérisé en ce que ce cathéter (30) comprend, en plus du tube de cathéter (12) et de la chambre de prélèvement d'échantillons (18), un ou plusieurs tube(s) supplémentaire(s) de cathéter (12) à chacun desquels est associée une chambre correspondante de prélèvement d'échantillons (18), tous les tubes (12) constituant le cathéter présentant diverses longueurs, si bien que les chambres respectives de prélèvement d'échantillons (18) sont disposées en des emplacements différents tout au long du cathéter (30), les parois de toutes ces chambres de prélèvement (18) étant réalisées dans la même matière.

3. Cathéter des revendications 1 ou 2, caractérisé en ce que le tube de cathéter (42) est constitué d'un tube rhinogastrique à reniflard et à double lumière avec une troisième lumière pour la chambre de prélèvement d'échantillons (18) à l'extrêmité de cette troisième lumière.

4. Cathéter des revendications 1 ou 2, caractérisé en ce que l'extrêmité (14) du tube de cathéter (12) comporte un sac de mercure fermé de façon étanche (52).

5. Cathéter des revendications 1 à 4, caractérisé en ce que la matière de la chambre de prélèvement d'échantillons (18) est un élastomère de polydiméthylsiloxane.

0 089 647

Fig. 1

Fig. 2

Fig. 3

Fig. 4

1